(19) Europäisches Patentamt

European Patent Office

Office européen des brevets

(11) Publication number: **0 093 837**
**B1**

# (12) EUROPEAN PATENT SPECIFICATION

(45) Date of publication of patent specification: **27.01.88**

(21) Application number: **83101469.1**

(22) Date of filing: **16.02.83**

(51) Int. Cl.⁴: **A 61 M 25/00**, A 61 M 1/28,
F 16 L 33/00

(54) A coupling for the connection of two flexible tubes or the like.

(30) Priority: **13.04.82 SE 8202290**

(43) Date of publication of application:
**16.11.83 Bulletin 83/46**

(45) Publication of the grant of the patent:
**27.01.88 Bulletin 88/04**

(84) Designated Contracting States:
**AT BE CH DE FR GB IT LI LU NL**

(56) References cited:
**WO-A-81/00053**
**GB-A-2 067 075**
**US-A-3 838 843**
**US-A-3 986 508**
**US-A-4 161 949**
**US-A-4 294 250**

(73) Proprietor: **Gambro Lundia AB**
**Box 10101**
**S-220 10 Lund (SE)**

(72) Inventor: **Christopherson, Kjell Hans Ingvar**
**Tamburinvägen 10**
**S-245 00 Staffanstorp (SE)**
Inventor: **Killmaier, Horst**
**Tiefenweg 23**
**D-7451 Hechingen-Boll (DE)**
Inventor: **Odelius, Tomas**
**Basfiolgränd 2**
**S-245 00 Staffanstorp (SE)**
Inventor: **Rosemeier, Friedrich**
**Boellatweg 1/1**
**D-7450 Hechingen (DE)**
Inventor: **Stenberg, Kaj Ove**
**Fugavägen 53**
**S-245 00 Staffanstorp (SE)**
Inventor: **Svensson, Per Tommy Emil**
**Ekelidsgatan 36**
**S-242 00 Hörby (SE)**

(74) Representative: **Boberg, Nils Gunnar Erik**
**Gambro AB Patent Department Box 10101**
**S-220 10 Lund (SE)**

Courier Press, Leamington Spa, England.

EP 0 093 837 B1

## Description

In accordance with the present invention a heat sterilizable connector comprising two mating connector elements for the connecting of two flexible tubes or the like is provided. At least one connector element is composed of two or more components of materials of different rigidity which components together withstand heat sterilization under pressure, the material of less rigidity being chosen to provide easy penetration and/or easy tearing. The connector is intended in the first place for the substantial sterile joining together of two medical components, such as e.g. a catheter to a flexible tube or a bag provided with a flexible tube.

Connectors of this type are used in connection with different medical treatments, e.g. extracorporeal treatment of blood or other body fluids. The connector in accordance with the present invention is intended first and foremost, however, to be used in connection with a method of treatment known under the name of CAPD (Continuous Ambulatory Peritoneal Dialysis). In this method of treatment a catheter is introduced by an operation into the patients' abdominal cavity and projects as such or via a flexible tube through the abdominal wall. This flexible tube should then be connected by a connector to a second flexible tube joined to a fluid bag. The connector should thus consist of two connector elements which are joined to the respective flexible tubes or possibly directly to the bag and/or the catheter. The first time such a connector is used, there is normally no problem, since both connector elements can be supplied in a sterilized condition. After a first usage however, when the fluid bag is to be replaced by a new one, at least the connector element connected to the abdominal catheter may be contaminated on its outside by external impurities. It will be readily understood that this may give rise to various complications.

Background Art

The connectors used at present are based primarily on the principle that the patient should not be able to touch the inner parts of the connector with his fingers. In this way it should be possible to keep them sterile. However, there is still a serious risk of contamination, if the outer components of the connector are polluted.

US—A—3 986 508 describes a connector of the abovementioned art, i.e. a connector comprising two mating connector elements for the connecting of two flexible tubes or the like, at least one connector element being composed of two or more components of materials of different rigidity which components together withstand heat sterilization under pressure. A disadvantage of this connector is that all components have to be made of materials which are resistant to high temperatures when the connector is intended to be heat sterilized under pressure. The choice of material is therefore restricted. Its ability for easy penetration or easy tearing cannot therefore be optimized.

US—A—4 161 949 describes another connector including thin membranes which seem to be rather easily penetrated. Also this connector has however to be made entirely from a material which is resistant to high temperatures. It is therefore a risk that the membranes have such a rigidity that they are cracked instead of teared providing a secondary risk of the creation of small particles in the liquid fed through the connector.

Disclosure of Invention

The connector in accordance with the present invention is characterized in that the material of lower rigidity is such that the component formed thereof, when heat sterilized alone under pressure, exhibits such dimensional alteration as to essentially affect the function of the connector, and in that the component (21a, 21b, 21c) of the material of higher rigidity is arranged to support the component (22a and/or 28a; 22b and/or 28b; 22c and or 28c) of the material of less rigidity against dimensional alteration during the heat sterilization.

This arrangement ensures for example, in CAPD, that at least the exchangeable connector element can be supplied completely sterile after heat sterilization in a condition in which it is easy penetrated and/or easily opened by the tearing away of a part of the same. At the same time, as will become evident from the following, a number of further advantages are obtained in connection with the handling of the connector.

For the sake of simplicity the various advantages of the invention will be described in the following in conjunction with its application to CAPD. It will be clear, however, to those versed in the art, that essentially the same advantages can be obtained in any other kind of connectors where it is desired to avoid contamination of the connector elements used.

Preferably, one of the connector elements is designed to have an outer component which is so rigid and thermally resistant that it withstands heat sterilization without any such dimensional alteration that could essentially affect the function of the connector, and an inner component provided with a membrane of said less rigid material, which on joining can readily be penetrated by a sharp component from the second connector element. Thanks to this combination, the construction can be such that the less rigid component too retains its dimensions during heat sterilization.

In a preferred embodiment one of the connector elements is designed so as to comprise a first component provided with an opening and being made of such a rigid and thermally resistant material that it withstands heat sterilization without any such dimensional alteration that could affect the function of the connector, and a second component closing the opening and being of said less rigid and therefore easily tearable material, and which is provided with a tearing indication making it possible for the part of the same to be torn off prior to the joining together of the two connector elements. This construction has also the advantage that the less rigid part can be manufactured from a type of material which otherwise would not withstand heat sterilization.

One component of the connector is preferably made up of an inner substantially cylindrical or tubular component of a non-rigid material, whose passage is blocked by a penetrable membrane, and of an outer component of a more rigid material wholly enclosing at least one opening of the inner component, which outer component projects outside said opening so that this opening is protected from being subjected to unintentional contact during the connection.

Appropriately both the inner component and the component enclosing it are provided with peripheral flanges for mutual joining together. These flanges may for example be substantially of the same outside diameter and be arranged plane surface against plane surface and enclosed by an outer connector component, e.g. a widened part of the flexible tube or pipe of which the connector element is intended to form an end piece. In such a construction the different components do not have to be manufactured from materials which are mutually weldable. However, the use of such materials is preferred, so that tightness may be assured further by welding.

The connector appropriately comprises a cylindrical component of a more rigid material in one of the connector elements which is provided with an internal thread adapted to an external thread or partial thread on a corresponding rigid cylindrical component in the other connector element, which is intended to be threaded into the firstnamed component during the connection. The said cylindrical component in the second connector element may consist here of a contact protection for a membrane-penetrating part in that it wholly encloses the latter. Owing to this construction the membrane-penetrating part as well as the membrane can be arranged in such a manner that any unintentional contact can safely be excluded.

Should any parts of the connector be contaminated, the danger of spreading of the contamination is reduced, if the above-mentioned membrane-penetrating part in the second connector element is adapted so that it engages in a tightly sealing manner with the component provided with the membrane in the first connector element before the membrane is penetrated.

The components of different rigidity are appropriately in such a positive engagement with one another that mutual turning is prevented when the connector elements are connected and/or when a part is teared away.

To ensure tightness still further, the said component which closes the opening and which consists of a less rigid and readily tearable material may be adapted so as to substantially enclose the whole of the connector element to which it belongs, so that it forms a tight seal at least with the flexible tube, pipe or the like of which the connector element is intended to form an end piece.

To ensure effective heat sterilization the above-mentioned membrane may be of a thickness which is such that steam can effectively diffuse through it during the sterilization. In this manner a sterilization even of any completely closed spaces on either side of the membrane is assured provided such steam has access to the other side.

The connector may also be provided with one or more auxiliary membranes which are not intended to be mechanically penetrated, but which have as their main objective to make possible the supply of steam to spaces which otherwise are closed during the heat sterilization.

Brief Description of the Drawings

In the following the invention will be described in greater detail with reference to the enclosed drawings which show some preferred embodiments of the subject of the invention.

Fig. 1 shows schematically the connector used in a CAPD system.

Fig. 2 shows a connector element in a first embodiment in closed condition.

Fig. 3 shows the same connector element partly opened.

Fig. 4 shows the same connector element, but here joined together with a second connector element.

Figs. 5 and 6 finally, show different modified embodiments of the connector element according to Fig. 2.

Brief Description of a CAPD System

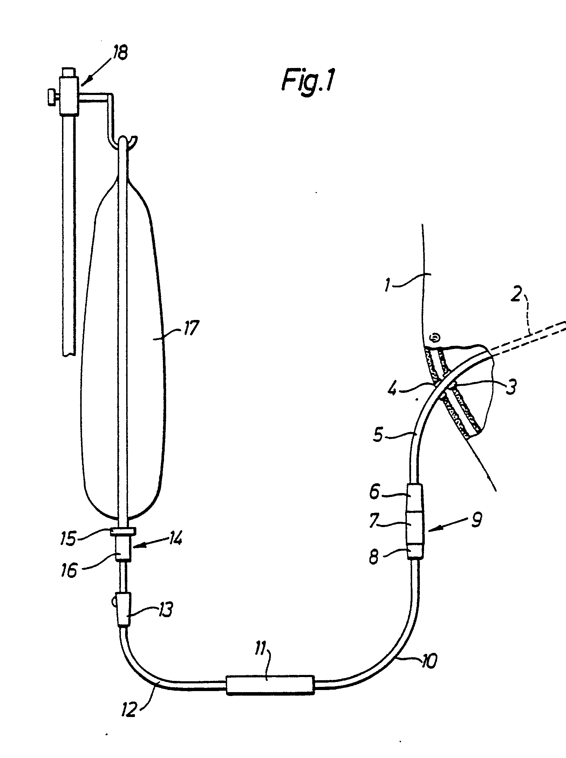

Fig. 1 shows schematically a CAPD system. Reference 1 designates the patient who in his abdomen has a catheter 2 introduced by operation which via two seals 3 and 4 passes over into an outer flexible tube 5. This tube is terminated by a connector element 6 which together with two connector elements 7 and 8 forms a connector 9 which is opened only infrequently. The connector element 8 is connected via a flexible tube 10 to a filter 11 which via a further flexible tube 12 with a tube clip 13 is joined to a connector 14 which is the actual subject of the present invention. By means of this connector 14, which consists of a first connector element 15 and a second connector element 16, a bag 17 filled with CAPD solution can be connected to the catheter 2 for the filling of the peritoneal cavity in the abdomen. In the example shown the bag is suspended on a simple stand 18.

A CAPD treatment thus consists in that the fluid from the bag 17 is introduced into the peritoneal cavity and is retained there for a number of hours, before it is emptied out again into the empty bag, jointly with any toxicant which it was meant to remove from the patient. After the emptying, the bag is disconnected together with the connector element 15, whereupon a new bag with a new connector piece 15 is connected for a repetition of the treatment.

Embodiment I

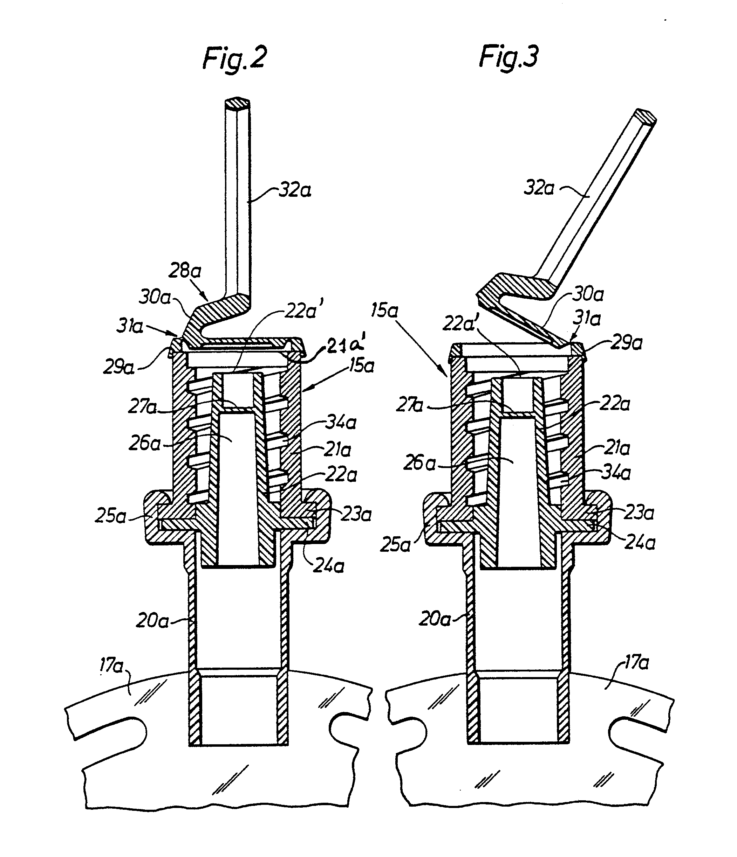

In Fig. 2 is shown a first embodiment of a connector element belonging to the connector in accordance with the invention. The top portion of a CAPD bag, designated 17a, is provided with an emptying nozzle 20a which may be said to constitute a part of a connector element referred to as a whole by 15a. This connector element 15a further comprises an outer cylindrical part 21a of a more rigid material and an inner, likewise cylindrical part 22a of a less rigid material. These two parts (defined as components in the claims) are provided with peripheral flanges 23a and 24a, respectively, which are preferably welded together apart from being enclosed by an outer, widened part 25a of the emptying nozzle 20a.

The passage 26a of the inner component 22a is blocked by a membrane 27a which can be readily penetrated by a sharp object, since the whole of the inner component 22a is made up of a relatively non-rigid material.

The top of the connector element is terminated by a component 28a closing the opening 21a' of the component 21a, which consists of a ring 29a welded to the component 21a and a tear-off part 30a which is attached to the ring 29a via a tearing indication 31a. The tearing is facilitated by means of a pull-ring or the like 32a.

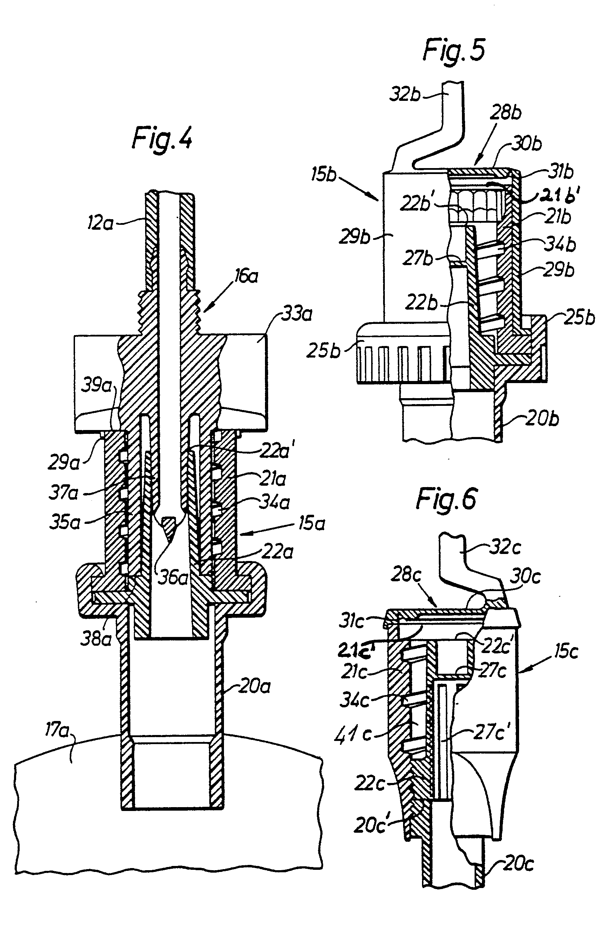

In Fig. 3 is shown the same connector element partly opened and in Fig. 4 it can be seen joined to a second connector element referred to as a whole by 16a. This connector element 16a is arranged as an end piece or an end portion on a flexible tube 12a. With the help of a finger grip 33a and an internal thread 34a on the component 21a, together with an external thread or partial thread (not shown) on its cylindrical portion 35a the connector element 16a can be screwed together with the connector element 15a whilst penetrating the membrane 27a. This penetration thus takes place with the help of the sharp component 36a at the front end of the inner plug 37a. The dimensions have been chosen so that the first seal is obtained at the outer opening 22a' of the inner component 22a, before the point 36a penetrates the membrane 27a. On the other hand and as a further safety, a seal is produced at 38a and 39a respectively, where the ring 29a is pressed together.

Embodiment II

In Fig. 5 a modified embodiment of the connector element according to Fig. 2 is shown. This embodiment includes substantially the same components as that according to Fig. 2. The same reference designations have been used, therefore, but the letter a has been replaced by b. The essential difference here is that the outer component 28b has been lengthened so that a cylinder 29b corresponding to the ring 29a extends down to the emptying nozzle 20b and is enclosed by the widened outer end 25b of the latter. This outer end is shown in this figure in its doubled back condition to the left and in its original condition to the right. For the rest the part 28b, as in the construction according to Fig. 2, comprises a pull ring 32b by means of which the part 30b can be torn off. The rest of the construction in accordance with Fig. 5 corresponds substantially to that according to Fig. 2 and does not, therefore, require any closer description.

Preferred Embodiment III

Fig. 6 finally shows a preferred embodiment of the subject of the invention. Here too the same references have been used but not the letters a and b, respectively have been replaced by c. In this embodiment the pipe nozzle 20c is welded to the inner component 22c at the front end 20c' of the pipe nozzle 20c. The outer rigid component 21c together with the welded-on component 28c has been forced subsequently over the inner component 22c and the outer end of the pipe nozzle 20c. Normally it would be sufficient here to provide a shrinkage fit, but if required glueing or welding may also be resorted to between these components.

As in the constructions according to Figs. 2 and 5 the top part 28c is provided with a pull ring 32c by means of which the part 30c can be removed.

An essential diference in respect of the embodiments described earlier is encountered in the thinner portions or membrane windows designated 27c' which are intended, similarly to the membrane 27c, to make possible a transfer of steam from the CAPD bag and its emptying nozzle 20c to the space 41c between the components 21c and 22c. By this arrangement an effective sterilization of this space in conjunction with the heat sterilization is assured.

Naturally it will be obvious to those versed in the art that a great number of available materials may be used for the different components. The following materials for example may be suggested: PVC for the components 20a, 20b, 20c, 22a, 22b, 22c. Polypropylene, polycarbonate or cellulose acetate for the components 21a, 21b and 21c. Polypropylene compounded with EVA for components 28a, 28b and 28c. Alternatively for the last mentioned components a suitable PVC quality may also be chosen.

Naturally the invention is not limited solely to the embodiments described above but can be varied within the scope of the following claims. For example those versed in the art have many other materials to choose from, and the individual components, moreover, can be varied within wide limits.

**Claims**

1. A heat sterilizable connector comprising one first and one second connector element for the connecting of two flexible tubes or the like, at least one of said connector elements (15, 15a, 15b, 15c) being composed of two or more components (21a, 22a, 28b; 21b, 22b, 28b; 21c, 22c, 28c) of materials of different rigidity which components together withstand heat sterilization under pressure, the material of less rigidity being chosen to provide easy penetration and/or easy tearing, characterized in that the material of lower rigidity is such that the component (22a and/or 28a; 22b and/or 28b; 22c and/or 28c) formed thereof, when heat sterilized alone under pressure, exhibits such dimensional alteration as to essentially affect the function of the connector and in that the component (21a, 21b, 21c) of the material of higher rigidity is arranged to support the component (22a and/or 28a; 22b and/or 28b; 22c and/or 28c) of the material of less rigidity during the sterilization.

2. A connector in accordance with claim 1, characterized in that one (21a, 21b, 21c) of said two or more components which is part of the first connector element and which is of a material of higher rigidity is an outer component which is mounted on an inner component (22a, 22b, 22c) of said less rigid material, said inner component being provided with a passage closed by a transverse membrane (27a, 27b, 27c) which can readily be penetrated by a sharp component (36a) forming part of the second connector element (16, 16a).

3. A connector in accordance with claim 1, characterized in that a first component (21a, 21b, 21c) of said material having a higher rigidity is provided at one end thereof with an opening (21a', 21b', 21c') closed by a second component (28a, 28b, 28c) of a less rigid and easily tearable material, said second component being provided with a tearing indication (31a, 31b, 31c) and with a tearable part (30a, 30b, 30c) to be torn off prior to the joining together of the two connector elements.

4. A connector in accordance with claim 2, characterized in that said inner component of less rigid material (22a, 22b, 22c) is of substantially tubular shape, and in that said outer component (21a, 21b, 21c) of a more rigid material is so shaped to wholly enclose at least one end opening (22a', 22b', 22c') of the inner component and to extend outwardly of said opening, to protect it from being subjected to unintentional contact during the connection of the two connector elements.

5. A connector in accordance with claim 4, characterized in that both the inner component (22a) and the outer component (21a) which form said first connector element are each provided with peripheral flanges (23a, 24a) adapted to be mutually joined together.

6. A connector in accordance with claims 5, characterized in that the said flanges (23a, 24a) are substantially of the same outside diameter and in that their planar surfaces opposite to their planar contacting surfaces are enclosed by a third outer component (25a), which is a widened end part of a flexible tube or a pipe (20a) of which the connector element is intended to form an end piece.

7. A connector in accordance with any one of the preceding claims, characterized in that said component (21a, 21b, 21c) of a more rigid material of the first connector elements is tubular and is provided with an internal thread (34a, 34b, 34c) adapted to cooperate with an external partial or complete thread formed on the external wall of a rigid tubular part (35a) of the second connector element, for screwing thereof into the first connector element.

8. A connector in accordance with claim 7, characterized in that the said tubular component (35a) of the second connector element (16a) comprises a protection sleeve surrounding an elongate membrane-penetrating part (37a).

9. A connector in accordance with claim 8, characterized in that the said membrane-penetrating part (37a) of the second connector element (16a) is adapted to tightly sealingly engage the internal wall of the inner component (22a) forming part of the first connector element (15a) provided with the membrane (27a) before penetration thereof.

10. A connector in accordance with any one of the preceding claims, characterized in that the components (20a, 21a, 22a, 28a) of different rigidity are in such positive engagement with one another that mutual turning is prevented when the connector elements are connected and/or when a part is torn away.

11. A connector in accordance with claim 3, characterized in that the component (28b) which closes the opening (22b') and which consists of a less rigid and therefore easily tearable material is so shaped as to substantially enclose most of the length of the connector element (15b) to which it belongs so that it forms a tight seal at least for a flexible tube or the pipe or the like (20b) of which the connector element is intended to form an end piece.

12. A connector in accordance with claim 2, characterized in that the said membrane (27a, 27b, 27c) is of a material and of a thickness such that steam can diffuse through it during heat sterilization.

13. A connector in accordance with claim 2, characterized in that the inner component (22c) of the first connector element is provided with one or more auxiliary membranes (27c') which are not intended to be mechanically penetrated but which are adapted to diffuse steam to closed spaces (41c) of the connector element (15c) which are not in communication with the outside heat sterilization.

**Patentansprüche**

1. Wärmesterilisierbares Verbindungsstück mit einem ersten und einem zweiten Verbinderelement für das Verbinden von zwei flexiblen Schläuchen oder dergleichen, wobei mindestens eines der Verbinderelemente (15, 15a, 15b, 15c) aus zwei oder mehr Bauteilen (21a, 22a, 28a; 21b, 22b, 28b; 21c, 22c, 28c) aus Materialien unterschiedlicher Steifheit zusammengesetzt sind, wobei die Bauteile zusammen der Wärmesterilisation unter Druck widerstehen, wobei das Material mit geringerer Steifheit ausgewählt wird, um ein leichtes Durchdringen und/oder ein leichtes Reißen vorzusehen, dadurch gekennzeichnet, daß das Material der geringeren Steifheit derart ist, daß das Bauteil (22a und/oder 28a; 22b und/oder 28b; 22c und/oder 28c), welches daraus gebildet ist, wenn es allein unter Druck wärmesterilisiert wird, solche Maßänderung zeigt, daß die Funktion des Verbindungsstückes wesentlich beeinflußt wird, und daß das Bauteil (21a, 21b, 21c) des Materials höherer Steifheit angeordnet ist, um das Bauteil (22a und/oder 28a; 22b und/oder 28b; 22c und/oder 28c) während der Sterilisation das Material geringerer Steifheit zu stützen.

2. Verbindungsstück nach Anspruch 1, dadurch gekennzeichnet, daß eines (21a, 21b, 21c) der zwei oder mehreren Bauteile, welches Teil des ersten Verbinderelementes ist und welches aus einem Material höherer Steifheit ist, ein äußeres Bauteil ist, welches auf einem inneren Bauteil (22a, 22b, 22c) des weniger steifen Materials angebracht ist, wobei das innere Bauteil mit einem Durchgang versehen ist, der durch eine Quermembran (27a, 27b, 27c) geschlossen ist, die leicht von einem scharfen Bauteil (36a) durchdrungen werden kann, welches Teil des zweiten Verbinderelementes (16, 16a) bildet.

3. Verbindungsstück nach Anspruch 1, dadurch gekennzeichnet, daß ein erstes Bauteil (21a, 21b, 21c) des Materials mit höherer Steifheit an seinem einen Ende mit einer Öffnung (21a', 21b', 21c') versehen ist, die von einem zweiten Bauteil (28a, 28b, 28c) aus einem weniger steifen und leicht reißbaren Material geschlossen ist, wobei der zweite Bauteil mit einer Reißanzeige (31a, 31b, 31c) und mit einem reißbaren Teil (30a, 30b, 30c) versehen ist, welches vor dem Zusammenfügen der zwei Verbinderelemente abzureißen ist.

4. Verbindungsstück nach Anspruch 2, dadurch gekennzeichnet, daß das innere Bauteil aus weniger steifem Material (22a, 22b, 22c) eine im wesentlichen rohrförmige Gestalt hat und daß das äußere Bauteil (21a, 21b, 21c) eines steiferen Materials so gestaltet ist, daß es vollständig wenigstens eine Endöffnung (22a', 22b', 22c') des inneren Bauteils einschließt und sich nach außerhalb der Öffnung erstreckt, um sie davor zu schützen, daß sie während der Verbindung der zwei Verbinderelemente einer unbeabsichtigten Berührung ausgesetzt wird.

5. Verbindungsstück nach Anspruch 4, dadurch gekennzeichnet, daß sowohl das innere Bauteil (22a) als auch das äußere Bauteil (21a), welche das erste Verbinderelement bilden, jeweils mit Umfangsflanschen (23a, 24a) versehen sind, die angepaßt sind, um gegenseitig zusammengefügt zu werden.

6. Verbindungsstück nach Anspruch 5, dadurch gekennzeichnet, daß die Flansche (23a, 24a) im wesentlichen denselben Außendurchmesser haben und daß ihre ebenen Oberflächen gegenüber ihren ebenen Berührungsoberflächen durch ein drittes äußeres Bauteil (25a) eingeschlossen sind, welches ein aufgeweitetes Endteil eines flexibien Schlauches oder eines Rohres (20a) ist, dessen Verbinderelement ein Endstück bilden soll.

7. Verbindungsstück nach einem der vorhergehenden Ansprüche, dadurch gekennzeichnet, daß das Bauteil (21a, 21b, 21c) aus einem steife-

ren Material der ersten Verbinderelemente rohrförmig ist und mit einem Innengewinde (34a, 34b, 34c) versehen ist, welches geeignet ausgestaltet ist, um mit einem äußeren teilweisen oder vollständigen Gewinde zusammenzuwirken, welches auf der Außenwand eines steifen, rohrförmigen Teils (35a) des zweiten Verbinderelementes gebildet ist, um es in das erste Verbinderelement einzuschrauben.

8. Verbindungsstück nach Anspruch 7, dadurch gekennzeichnet, daß das rohrförmige Bauteil (35a) des zweiten Verbinderelementes (16a) eine Schutzhülse aufweist, welche ein längliches, membrandruchdringendes Teil (37a) umgibt.

9. Verbindungsstück nach Anspruch 8, dadurch gekennzeichnet, daß das membrandurchdringende Teil (37a) des zweiten Verbinderelementes (16a) geeignet derart ausgestaltet ist, daß es dichtend mit der Innenwand des inneren Bauteils (22a) in Eingriff tritt, welches Teil des ersten Verbinderelementes (15a) bildet, welches vor seinem Durchdringen mit der Membran (27a) versehen ist.

10. Verbindungsstück nach einem der vorhergehenden Ansprüche, dadurch gekennzeichnet, daß die Bauteile (20a, 21a, 22a, 28a) unterschiedlicher Steifheit in derart wirksamem Eingriff miteinander stehen, daß ein gegenseitiges Drehen verhindert wird, wenn die Verbinderelemente verbunden werden und/oder wenn ein Teil abgerissen wird.

11. Verbindungsstück nach Anspruch 3, dadurch gekennzeichnet, daß das Bauteil (28b), welches die Öffnung (22b') verschließt und welches aus einem weniger steifen und deshalb leicht reißbaren Material besteht, so ausgestaltet ist, daß im wesentlichen das meiste der Länge des Verbinderelementes (15b) eingeschlossen wird, zu welchem es gehört, so daß es eine Dichtung mindestens für einen flexiblen Schlauch oder das Rohr oder dergleichen (20b) bildet, dessen Verbinderelement ein Endstück bilden soll.

12. Verbindungsstück nach Anspruch 2, dadurch gekennzeichnet, daß die Membran (27a, 27b, 27c) aus einem Material und einer Dicke derart besteht, daß Dampf während der Wärmesterilisation sich durch es ausbreiten kann.

13. Verbindungsstück nach Anspruch 2, dadurch gekennzeichnet, daß das innere Bauteil (22c) des ersten Verbinderelementes mit einer oder mehreren Hilsmembranen (27c') versehen ist, die nicht mechanisch durchdrungen werden sollen, die aber geeignet so ausgestaltet sind, daß sich Dampf zu geschlossenen Räumen (41c) des Verbinderlementes (15c) ausbreitet, welche während der Wärmesterilisation nicht mit der Außenseite in Verbindung stehen.

**Revendications**

1. Raccord stérilisable par la chaleur, comprenant un premier et un deuxième éléments de raccord pour la connexion de deux tubes flexibles ou analogues, au moins un des dits éléments de raccord (15, 15a, 15b, 15c) étant constitué de deux composants ou plus (21a, 22a, 28a; 21b, 28b; 21c, 22c, 28c) en matières de rigidité différente, ces composants assemblés supportant la stérilisation à chaud sous pression, la matière de plus faible rigidité étant choisie pour permettre une pénétration facile et/ou une déchirure facile, caractérisé en ce que la matière de plus faible rigidité est telle que le composant (22a et/ou 28a; 22b et/ou 28b; 22c et/ou 28c) fabriqué en cette matière, lorsqu'il est stérilisé à chaud seul et sous pression, subit un changement dimensionnel qui affecte sensiblement le fonctionnement du raccord, et en ce que le composant (21a, 21b, 21c) en matière de plus grande rigidité est agencé de manière à supporter le composant (22a et/ou 28a, 22b et/our 28b; 22c et/ou 28c) en matière de plus faible rigidité, pendant la stérilisation.

2. Raccord suivant la revendication 1, caractérisé en ce que l'un (21a, 21b, 21c) desdits deux composants ou plus, qui fait partie du premier élément de raccord et qui est en une matière de plus grande rigidité, est un composant extérieur qui est monté sur un composant intérieur (22a, 22b, 22c) en dite matière moins rigide, ledit composant intérieur comportant un passage fermé par une membrane transversale (27a, 27b, 27c) qui peut facilement être pénétrée par un composant pointu (36a) faisant partie du deuxième élément de raccord (16, 16a).

3. Raccord suivant la revendication 1, caractérisé en ce qu'un premier composant (21a, 21b, 21c) en ladite matière de plus grande rigidité comporte à une de ses extrémités un orifice (21a', 21b', 21c') fermé par un deuxième composant (28a, 28b, 28c) en une matière moins rigide et plus facilement déchirable, ledit deuxième composant comportant une indication de déchirure (31a, 31b, 31c) et une partie déchirable (30a, 30b, 30c) à déchirer avant l'assemblage des deux éléments de raccord.

4. Raccord suivant la revendication 2, caractérisé en ce que ledit composant intérieur en matière moins rigide (22a, 22b, 22c) est de configuration sensiblement tubulaire et en ce que ledit composant extérieur (21a, 21b, 21c) en matière plus rigide est d'une configuration telle qu'il entoure complètement au moins un orifice d'extrémité (22a', 22b', 22c') du composant intérieur et qu'il se prolonge à l'extérieur dudit orifice afin de protéger celui-ci contre l'exposition à un contact involontaire pendant le raccordement des deux éléments de raccord.

5. Raccord suivant la revendication 4, caractérisé en ce que le composant intérieur (22a) et le composant extérieur (21a) qui constituent ledit premier élément de raccord comportent chacun des collerettes périphériqués (23a, 24a) prévues pour être mutuellement reliées.

6. Raccord suivant la revendication 5, caractérisé en ce que lesdites collerettes (23a, 24a) ont sensiblement le même diamètre extérieur et en ce que leurs surfaces planes, opposées à leurs surfaces planes en contact, sont entourées par un troisième composant extérieur (25a), qui est une partie d'extrémité élargie d'un tube flexible ou d'un tuyau (20a) dont l'élément de raccord est destiné à former une pièce d'extrémité.

7. Raccord suivant l'une quelconque des revendications précédentes, caractérisé en ce que ledit composant (21a, 21b, 21c) en matière plus rigide du premier élément de raccord est tubulaire et comporte un filetage intérieur (34a, 34b, 34c) prévu pour coopérer avec un filetage extérieur partiel ou complet formé sur la paroi extérieure d'une partie tubulaire rigide (35a) du deuxième élément de raccord, pour vissage de celui-ci dans le premier élément de raccord.

8. Raccord suivant la revendication 7, caractérisé en ce que ledit composant tubulaire (35a) du deuxième élément de raccord (16a) comprend un manchon de protection entourant une partie allongée (37a) de pénétration de membrane.

9. Raccord suivant la revendication 8, caractérisé en ce que ladite partie de pénétration de membrane (37a) du deuxième élément de raccord (16a) est prévue pour venir en contact d'étanchéité serré avec la paroi intérieure du composant intérieur (22a), faisant partie du premier élément de raccord (15a) et comportant la membrane (27a), avant la pénétration dans cette membrane.

10. Raccord suivant l'une quelconque des revendications précédentes, caractérisé en ce que les composants (20a, 21a, 22a, 28a) de rigidité différente sont en contact positif les uns avec les autres de sorte qu'une rotation mutuelle est empêchée lorsque les éléments de raccord sont assemblés et/ou lorsqu'on déchire une partie.

11. Raccord suivant la revendication 3, caractérisé en ce que le composant (28b), qui ferme l'orifice (22b') et qui est en une matière moins rigide et donc facilement déchirable, a une configuration telle qu'il entoure sensiblement la plus grande partie de la longueur de l'élément de raccord (15b) auquel il appartient, de façon à engendrer une fermeture étanche au moins pour un tube flexible ou un tuyau ou analogue (20b) dont l'élément de raccord est prévu pour constituer une pièce d'extrémité.

12. Raccord suivant la revendication 2, caractérisé en ce que ladite membrane (27a, 27b, 27c) est en une matière et d'une épaisseur telles que la vapeur peut diffuser à travers la membrane pendant la stérilisation à chaud.

13. Raccord suivant la revendication 2, caractérisé en ce que le composant intérieur (22c) du premier élément de raccord comporte une ou plusieurs membrances auxiliaires (27c') qui ne sont pas destinées à être pénétrées mécaniquement mais qui sont prévues pour diffuser la vapeur aux espaces fermées (41c) de l'élément de raccord (15c) qui ne communiquent pas avec l'extérieur, pendant la stérilisation à chaud.

Fig.1

Fig.2    Fig.3

## Fig.4

## Fig.5

## Fig.6